# EUROPEAN PATENT APPLICATION

(11) **EP 2 462 952 A1**
(43) Date of publication of application: **13.06.2012**
(21) Application number: 10194621.8
(22) Date of filing: 10.12.2010
(51) Int. Cl.: A61K 48/00, A61K 38/16

(54) **Cancer gene therapy using nucleic acids encoding US28**

(71) Applicant: Medizinische Universität Graz, 8010 Graz (AT)
(72) Inventor: Schaider, Helmut, 8010, Graz (AT); Joshi, Shripad, 8043, Graz (AT)
(74) Representative: Bühler, Dirk

(57) **Abstract**

The invention relates to the gene therapeutic treatment of cancer using co-expressed nucleic acids encoding US28 and a G-protein, e.g. GNA-13, or functional fragments thereof, or using nucleic acids encoding fusion polypeptides of US28 and a G-protein or functional fragments thereof The pharmaceutical compositions according to the invention are used in the treatment of cancer patients to induce apoptosis in tumor cells.

## Description

### FIELD OF THE INVENTION

The present invention relates to the field of cancer gene therapy using nucleic acid molecules encoding US28 and G-proteins, respectively, or functional derivatives thereof, for example functional fragments thereof In the cancer gene therapy according to the invention nucleic acid molecules encoding fusion proteins of the above proteins or functional derivatives thereof, for example functional fragments thereof may also be used. The invention further relates to pharmaceutical compositions comprising the above nucleic acid molecules as well as methods or therapeutic uses of said nucleic acids or pharmaceutical compositions for inhibiting, retarding, ameliorating, and/or treating cancer.

### BACKGROUND OF THE INVENTION

Cancer is one of the leading causes of death and is responsible for increasing health costs. Traditionally, cancer has been treated using chemotherapy, radiotherapy and surgical methods. Tumour cell plasticity and heterogeneity, however, remain challenges for effective treatments of many cancers. Traditional therapies may have drawbacks, e.g. insufficient specificity, intolerable toxicity and too low efficacy.

In recent years molecular therapies have been developed, which eliminate cancer cells and prolong the survival time of affected patients or to cure said patients. One area of interest in the field of cancer therapy is directed to the induction of apoptosis in cancer cells. Frequently, constitutive activation of signalling pathways in those cells results in the induction of resistance to apoptosis. To target and reverse anti-apoptotic mechanisms is attractive and an example of molecularly-targeted therapy. For example, members of the *bcl-2* gene family and the "inhibitor of apoptosis protein"- families have been successfully targeted to render tumour cells more susceptible to apoptosis.

In previous attempts to induce apoptosis in cancer cells, pro-apoptotic fusion polypeptides have also been provided. For example, Samel et al., Journal of Biological Chemistry, 2003, Vol. 278, No. 34, 32077-32082, describe fusion polypeptides consisting of the pro-apoptotic protein FasL and a fibroblast activation protein (FAP)-specific single chain antibody fragment (sc40-FasL) that prevented growth of xenotransplanted FAP-positive (but not FAP-negative) tumour cells upon intravenous application.

In Bertin et al., Int. J. Cancer, 1997, Vol. 71, 1029-1034, the use of a fusion protein comprising human β2-adrenergic receptor and GS-alpha in the treatment of ras-dependent murine carcinoma cell lines in the prevention of tumour growth in syngeneic mice is disclosed.

WO 00/11950 discloses an assay system for determining therapeutic activity for treating restenosis, atherosclerosis, chronic rejection syndrome and graft versus host disease (GVHD) by measuring inhibition of cell migration activity in smooth muscle cells expressing a US28 receptor from the CMV genome.

WO 02/17900 describes assays, compositions and methods of treatment for modulating the binding of chemokines to US28 on the surface of cells.

US 2008/0020994 Al discloses agents that reduce expression of Gα12 or Gα13 polypeptides and contemplates their use in anti-cancer screening methods.

Pleskoff et al., FEBS Journal 272 (2005), 4163-4177, describe the effect of human cytomegalovirus-encoded chemokine receptor US28 on caspase-dependent apoptosis.

The advent of molecularly-targeted therapy raised hopes that therapeutics tailored, e.g. to specifically affect single molecules crucial to tumour biology provide effective and potentially less or non-toxic measures for a broad range of cancers. However, due to tumour plasticity and other factors influencing tumour growth and progression, e.g. the host response, tumour angiogenesis or the tumour microenvironment, the targeting of single molecules, even in combination with traditional therapeutics, may be insufficient to obtain sustainable effects resulting in survival prolongation or cure. Accordingly, there is a constant need to provide new and improved cancer therapies.

The present invention relates to gene therapeutic methods involving the co-expression of proteins as a result of successful transduction or transfection of tumor cells with nucleic acid molecules. These nucleic acids may code for the protein US28 and at least one G-protein (subunit) such as GNA12 or GNA13. Alternatively, functional fragments of US28 and the at least one G-protein or, in a further alternative, fusion proteins of US28 and G-proteins or fusion polypeptides of functional fragments thereof are expressed in tumor cells which have previously been transduced or transfected with nucleic acids encoding the same. Additionally, in the gene therapeutic methods of the present invention chimaeric nucleic acid molecules comprising functionally important fragments of the G-proteins referred to herein may be used. The fusion proteins of the present invention may encode US28 or a functional derivative thereof and a chimaeric G-protein that comprises fragments or domains derived from different G-proteins, e.g. parts of GNA12 and GNA13, respectively.

Co-expression of the polypeptides disclosed herein activates genes and/or proteins that are involved in apoptosis and/or reduces proliferation in respective cells. Therefore, the nucleic acids used in the context of the invention, compositions comprising the same and uses thereof provide tools for hitherto unknown and surprisingly effective methods of treating cancer or alleviating symptoms that are caused by cancer.

### DEFINITIONS

Before the present invention is described in more detail, definitions of various terms used hereinbelow are provided.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

The term "carrier" is used herein to refer to a pharmaceutically acceptable vehicle for a pharmacologically active agent. The carrier facilitates delivery of the active agent to the target site without terminating the function of the agent. Non-limiting examples of suitable forms of the carrier include solutions, creams, gels, gel emulsions, jellies, pastes, lotions, salves, sprays, ointments, powders, solid admixtures, aerosols, emulsions (e.g., water in oil or oil in water), gel aqueous solutions, aqueous solutions, suspensions, liniments, tinctures, and patches suitable for topical administration.

The term "effective" is used herein to indicate that the nucleic acids used in the context of the present invention are administered in an amount and at an interval that results in the induction of apoptosis or arrest or slower proliferation of cancer cells. The induction of apoptosis may result, inter alia, in the reduction of tumour size or tumour volume, prevention of formation of metastases, inhibition or prevention of neovascularization of tumor tissues, et cetera. In clinical terms, an effective treatment means that a complete response or a partial response is achieved.

Within the context of the present invention, "gene therapy" designates the use of nucleic acid molecules or compositions comprising the same in the treatment of a patient in need thereof The nucleic acid molecules and compositions comprising the same are used in the treatment of cancer patients, wherein cancer cells are transfected or transduced with said nucleic acid molecules. As a consequence of the transfection, these cells express the encoded protein(s). Expression of the nucleic acids and/orproteins leads to the induction of apoptosis in cancer cells. Gene therapeutic methods according to the invention may rely on viral or non viral methods as explained below.

The terms "nucleic acid(s) of the invention" or "nucleic acid molecule(s) of the invention" used herein designate a sequence of nucleotides that may be used per se or in the compositions or methods described herein. The terms refer to the entire coding sequence of the US28 and G-proteins, respectively, mentioned herein. Furthermore, the terms also designate nucleic acids encoding functional protein fragments, vectors comprising the coding sequences or functional fragments of the above proteins as well as derivatives of the nucleic acids referred to herein, which have modifications, i.e. deletions, additions, inversions, etc. of one or more, e.g. 1 to 50, 1 to 40, 1 to 30, 1 to 20, 1 to 10, 1 to 5, 1 to 3, 2, or 1 nucleotide(s), which nevertheless encode polypeptides that are capable of activating apoptosis and/or preventing the proliferation of tumor cells. Functional derivatives of the nucleic acids of the invention encode polypeptides that induce at least 10%, 20%, 30%, 40%, 50%, 60%, 70%, 80%, 90% or more apoptosis under conditions described herein, i.e. upon co-expression in cancer cells, when compared with the wild-type polypeptide. Further derivatives of the nucleic acids encoding G-proteins may be chimaeric nucleic acid molecules encoding chimaeric G-proteins, e.g. G-proteins that comprise parts or domains of different G-proteins. For example, a chimaeric G-protein may be encoded by nucleic acid molecules that code for parts or domains of GNA12 and GNA13, respectively. Moreover, as indicated above, the terms "nucleic acid(s) of the invention" or "nucleic acid molecule(s) of the invention" designate also vectors comprising the nucleic acids described herein. These vectors may contain regulatory sequences allowing for the efficient transcription of the herein described nucleic acids.

Within the context of the present invention, the terms "functional fragment" or "functional derivative" mean that partial nucleic acid sequences of the entire nucleic acid sequences encoding US28 and/or the G-protein interacting herewith, nucleic acids encoding fusion proteins or fragments thereof and nucleic acids that code for chimaeric G-proteins (also as parts of a fusion protein of the invention) may be used as long as a sufficient quantity ofprotein(s) is expressed to activate at least about 10%, 20%, 30%, 40%, 50%, 60%, 70%, 75%, 80%, 90%, 95%, or 100% or even a higher percentage of apoptosis in cancer cells subsequent to the transduction or transfection with the nucleic acid molecules of the invention, e.g. within the next 1, 2, 3, 4, 5, or 6 months, the next one, two, three, or four weeks, alternatively within 120, 108, 96, 72, 48, 24, or 12 hours, when compared with the percentage of cells undergoing apoptosis that have been successfully transfected with the entire coding sequence of US28 and the G-protein, e.g. GNA13 or GNA12. Furthermore, the terms also mean that the proliferation of tumor cells that have been subjected to the gene therapy of the invention is reduced or slowed down, e.g. the proliferation of comparative numbers of tumor cells that have been subjected to the gene therapy of the invention, as compared with tumor cells that have not been subjected to gene therapy, is reduced by at least 10%, 20%, 30%, 40%, 50%, 60%, 70%, 75%, 80%, 90%, 95%, or 100% in said treated cells. Methods for the determination of cells numbers are known in the art, e.g. using automatic cell counters.

Within the context of the present invention, "sufficient quantity ofprotein(s)" means that substantially all of the cancer cells expressing said protein(s) that are encoded by the nucleic acid molecules of the invention or at least about 10%, 20%, 30%, 40%, 50%, 60%, 75%, 90% or 95% or even a larger number of the transfected or transduced cells expressing said protein(s) undergo apoptosis subsequent to the gene therapeutic treatment, e.g. within the next months, the next one, two, three, or four weeks, alternatively within 120, 108, 96, 72, 48, or 24 hours.

### BRIEF DESCRIPTION OF THE FIGURES

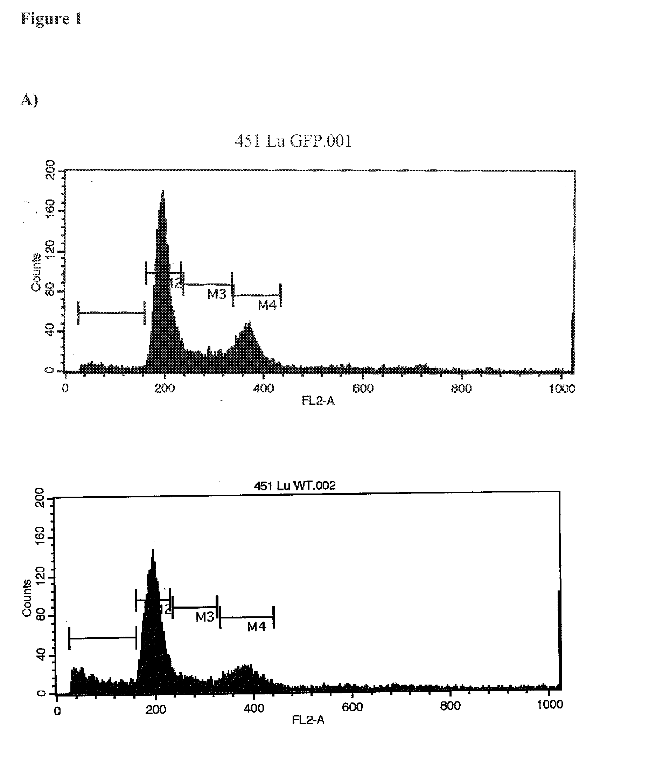

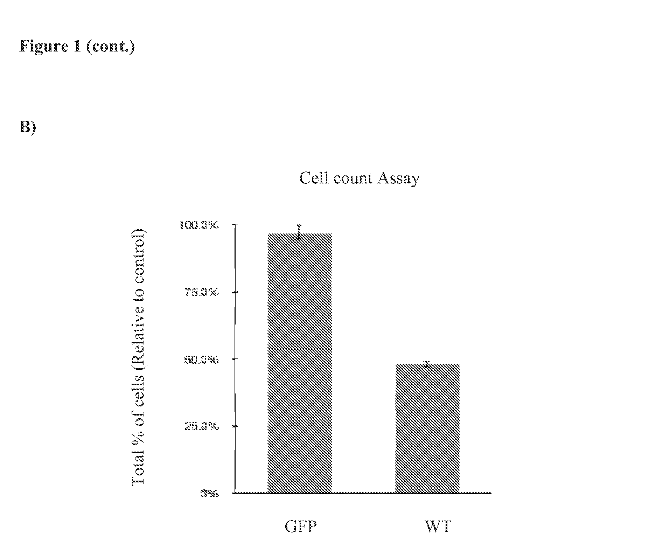

- Figure 1: Effect of US28 expression in melanoma cells. 451Lu cells transiently transfected with the plasmids pcDNA3.1-GFP and pcDNA3.1-US28 wild type (WT) were analyzed at 72 hrs post transfection to determine apoptosis stimulated DNA fragmentation by FACS (Fig. 1A). The cell number was determined at 72 hrs post-transfection (Fig. 1B).

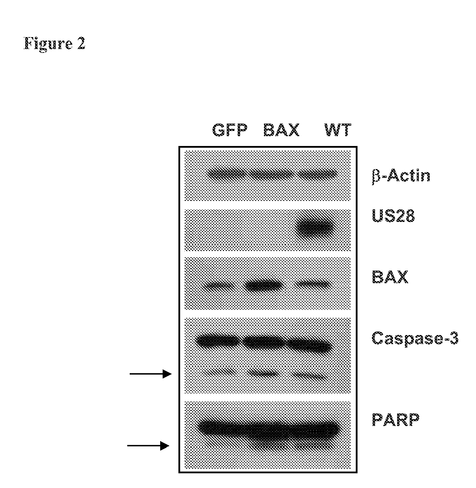

- Figure 2: US28 induces caspase mediated apoptosis in 451Lu cells transfected with plasmids encoding US28 and positive control BAX but not in cells transfected with control GFP. An SDS-PAGE of the proteins harvested at 72 hrs post transfection is shown. Arrows indicate the cleaved products due to activation of apoptotic pathways. There was a significant increase in activated caspase-3 upon transfection with BAX or US28. An increase was also observed for the cleavage product of PARP, the indicator target of activated caspases, due to US28 expression detected at 72 hrs post-transfection.

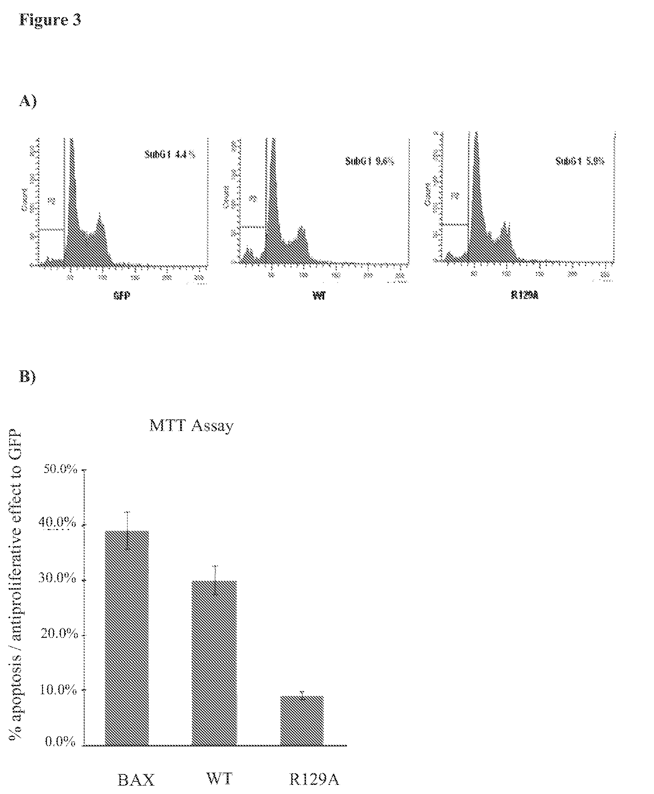

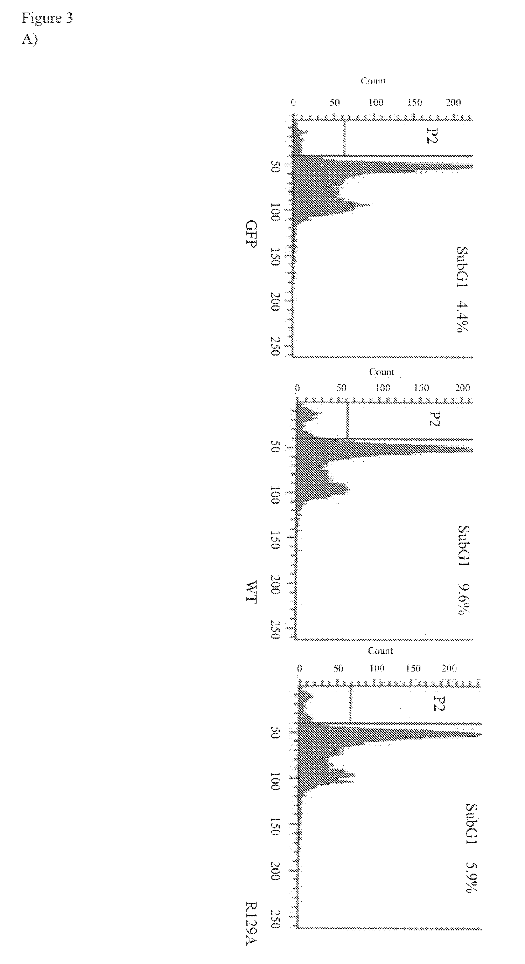

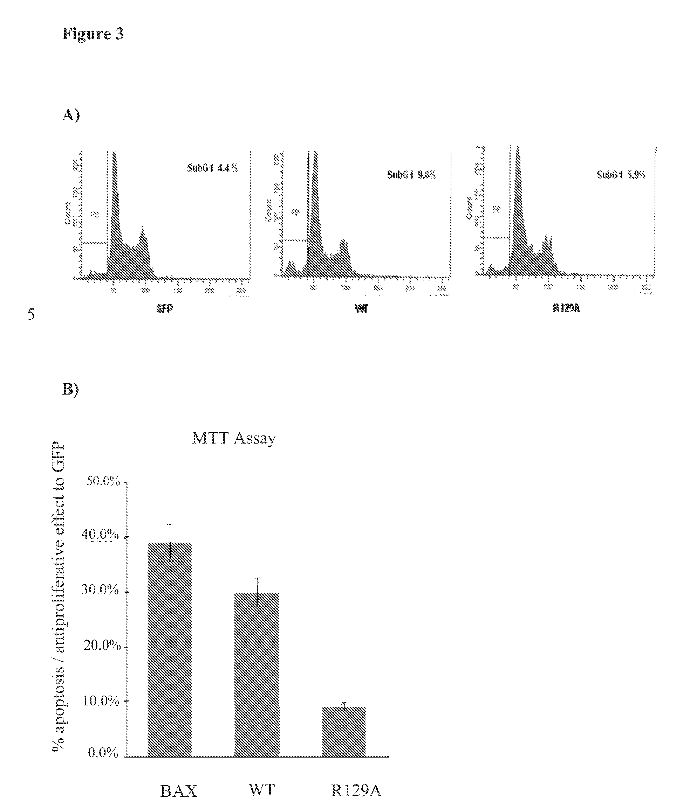

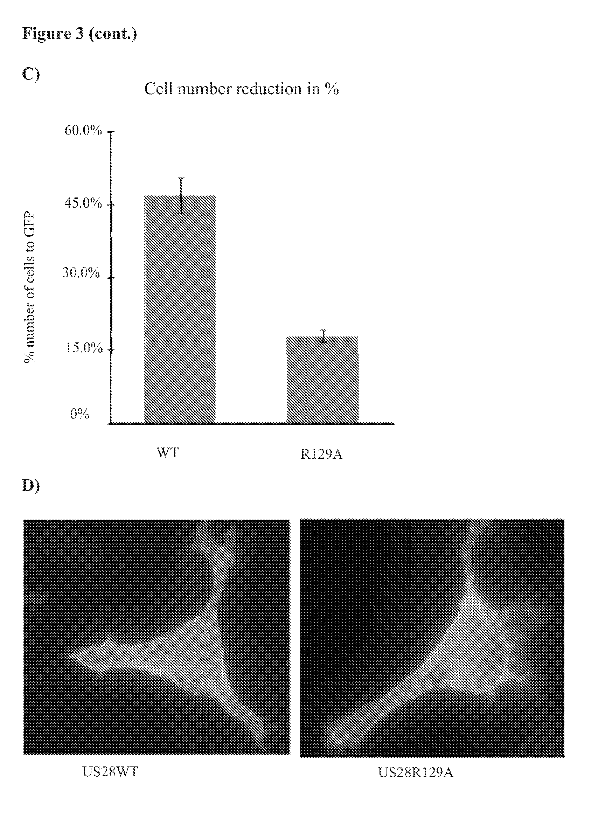

- Figure 3: Signaling of US28 is crucial for apoptotic/antiproliferative effects. 451Lu cells transfected with US28WT and US28R129A were analyzed at 72 hrs post transfection. (A) For comparative apoptotic DNA fragment cells transfected with GFP, WT and R129A were subjected to FACS-analysis. (B) Transfected 451Lu melanoma cells analyzed at 72 hrs after transfection by MTT assay kit. (C) Cell count performed at 72 hrs with transfected 451Lu cells. (D) Expression of US28 was confirmed by immunostaining procedure. Green/bright fluorescence is found at the plasma membrane.

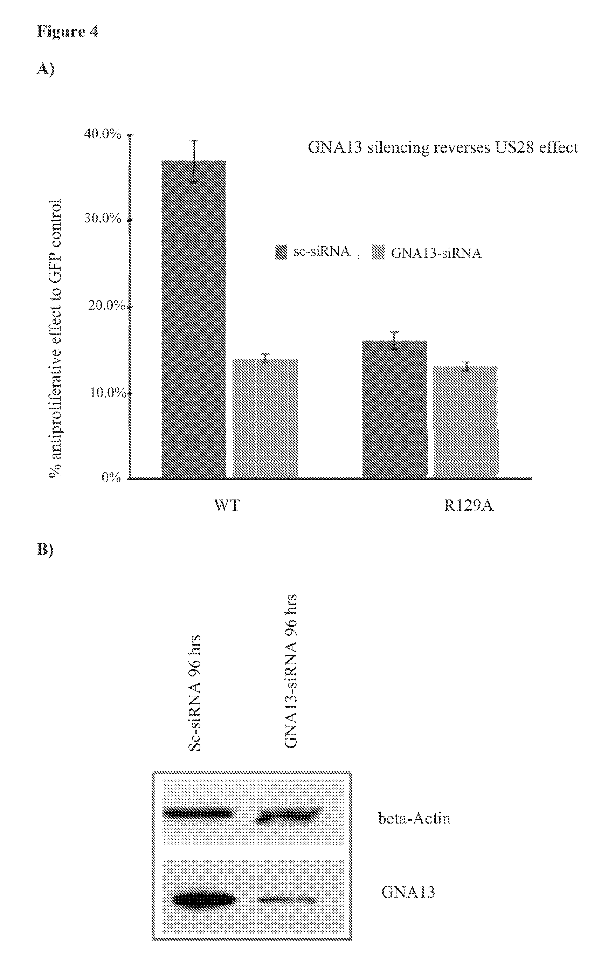

- Figure 4: Role of GNA13 in US28 mediated apoptotic/anti-proliferative effect, 451Lu cells were silenced for expression of GNA13 by using siRNA, followed by transfection with US28 WT and R129A constructs along with GFP control. The transfected cells were analyzed at 72 hrs after transfection by MTT assay kit and colorimetric measurements were performed. Reversal of the US28 effect in the case of GNA13 if compared to scrambled siRNA silencing experiments (Fig. 4A). Immunoblotting confirmed silencing of GNA13 as visualized by a nearly complete downregulation of GNA13 up to 96 hrs (Fig. 4B).

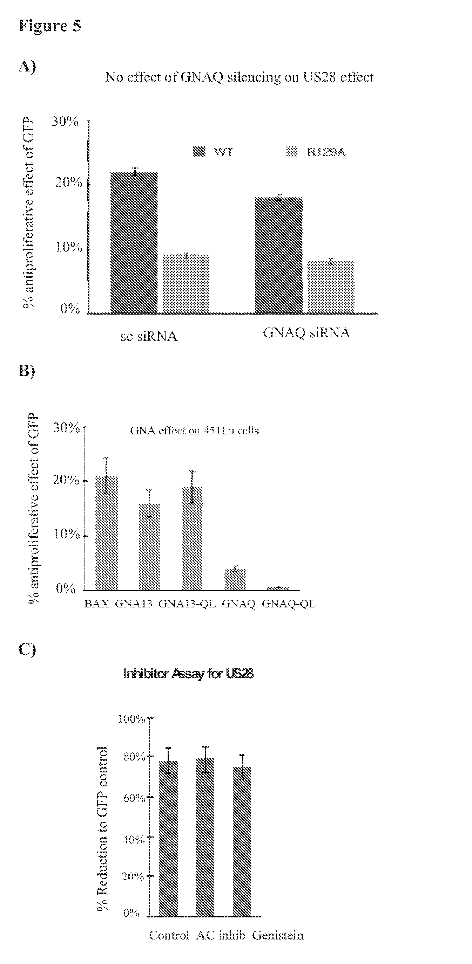

- Figure 5: Investigation of role of other potential G-protein complexes interacting with US28 by experiments involving either G-protein pathway specific inhibitor (Adenylate cyclase inhibitor for GNAS G-protein) and by silencing (GNAQ siRNA).

### DETAILED DESCRIPTION

Apoptosis is the process of programmed cell death that may occur in multicellular organisms. Biochemical events in the cells lead to characteristic morphologic changes and finally to cell death. Morphologic changes include membrane blebbing, loss of cell membrane asymmetry and substrate attachment, cell shrinkage, nuclear fragmentation, chromatin condensation, and chromosomal DNA fragmentation. Apoptosis may be triggered by developmental factors resulting in controlled growth, but may also be stimulated through external influences such as infectious agents, chemical noxes etc. In normal tissues, apoptosis plays a role in tissue homeostasis. In tumours, apoptosis is frequently deregulated resulting in uncontrolled proliferation of the tumour tissue. Apoptotic pathways are frequently suppressed in tumours, thereby avoiding cell death with a subsequent increase of tumour cell numbers and an increase of tumour volume.

The use in cancer gene therapy methods of nucleic acid molecules encoding US28 and G-proteins such as GNA12 or GNA13, or the use of nucleic acid molecules encoding functional fragments or derivatives thereof, or, in yet another alternative, the use of nucleic acid molecules encoding a fusion polypeptide (also referred to as fusion protein) of US28 and a G-protein, e.g. GNA13 or GNA12, or of functional fragments thereof provides a new and effective tool to destroy tumour cells via activation of processes leading to apoptosis.

US28 designates an open reading frame found in the genome of human cytomegalovirus (HCMV). US28 encodes a protein containing seven putative membrane-spanning domains, and a series of well-defined sequence motifs characteristic of the rhodopsin-like G-protein-coupled receptor family. US28 is related to a capripoxvirus gene that encodes a protein with features of members of the G-protein-coupled receptor subfamily (reference is made, e.g. to Horst Ibelgauft's Cytokines & Cells Online Pathfinder Encyclopedia, http://www.copewithcytokines.de/cope.cgi?key=US28). G-proteins (guanine nucleotide-binding proteins) form a family of proteins involved in transmitting chemical signals outside the cell, and causing changes inside the cell. They communicate signals from many hormones, neurotransmitters, and other signalling factors. G-proteins regulate metabolic enzymes, ion channels, transporters, and other parts of the cell machinery, controlling transcription, motility, contractility, and secretion, which in turn regulate systemic functions such as embryonic development, learning and memory, and homeostasis. Receptor-activated G proteins are bound to the inside surface of the cell membrane. They consist of the Gα and the tightly associated Gβγ subunits. There are four classes of Gα subunits: Gas, Gai, Gαq/11, and Gα12/13. They behave differently in the recognition of the effector, but share a similar mechanism of activation. Gα12/13 are involved in Rho family GTPase signalling (through the RhoGEF superfamily) and are involved in the control e.g. cell cytoskeleton remodelling and cell migration.

### Nucleic acids/proteins

In one aspect of the present invention, expression of US28 activates cellular processes that lead to apoptosis and diminishes proliferation in highly aggressive tumour cells upon interaction with proteins belonging to the G-protein family such as GNA13 and or GNA12.

Accordingly, the use of nucleic acid molecules encoding US28 or functional fragments thereof in combination with functional partners of said protein in gene therapy is a new and beneficial way to reduce the number of cancer cells and thereby treat cancer, prolong the survival of affected patients, and improve the quality of life of such cancer patients.

In a further aspect, the present invention relates to nucleic acid molecules or nucleic acid constructs, e.g. vectors, encoding a novel fusion protein comprising US28 or a functional fragment thereof and nucleic acid molecules or nucleic acid constructs encoding a G-protein or functional fragment thereof, for example GNA13 and/or GNA12 or a functional fragment thereof These nucleic acid molecules are suitable for use in cancer gene therapy. The encoded proteins are capable of activating apoptosis-inducing genes and pro-apoptotic polypeptides in target cells, i.e. cancer cells.

The nucleic acid sequences of the present invention are capable of expressing gene products and the polypeptides in target cells, i.e. cancer cells when they are used under appropriate expression conditions to activate apoptosis-inducing genes and pro-apoptotic polypeptides.

In another aspect, the present invention relates to a fusion protein encoded by the herein described nucleic acid molecules.

In a specific aspect, the invention provides nucleic acid molecules that comprise the nucleotide sequences of SEQ ID NO: 1 (encoding US28) and/or SEQ ID NO: 3 (encoding GNA13) or nucleic acids that comprise functional fragments or functional derivatives thereof. These nucleotide sequences, functional fragments or functional derivatives are capable of activating genes and pro-apoptotic polypeptides and thereby induce apoptosis and/or reduce proliferation of cancer cells.

The invention further relates to nucleic acid molecules that comprise variations or mutations in the nucleic acid sequences of the invention as long as their capacity to induce (pro)-apoptotic functions in target cells is preserved.

According to one aspect, the invention provides a nucleic acid, which may encode a fusion protein, comprising, consisting essentially of, or consisting of nucleotide sequences respectively having at least about 50%, 60%, 70%, 80%, 90%, 95% or a higher percentage identity to the fused nucleotide sequences depicted e.g. in SEQ ID NO: 1 and SEQ ID NO: 3, respectively, or fused fragments or derivatives of these sequences inducing apoptosis in tumour cells. In some embodiments, the nucleotide sequences have at least about 85%, at least about 90%, at least about 95%, at least about 99%, or 100% identity to the nucleotide sequences of the respective partial sequences encoding the fusion proteins (e.g. US28 and GNA13 or GNA12) and are capable of activating genes and pro-apoptotic polypeptides that induce apoptosis in tumour cells expressing the same.

In a further embodiment, a nucleic acid molecule of the invention further comprises one or more nucleotide sequences regulating gene activity, e.g., promoters, which may be natural promoters of the genes used herein or exogenous promoter operably linked to the genes used herein. The promoter sequences may be derived from other genes as well as artificial promoters such as chimeric promoters that combine nucleic acid sequences derived from various sources, i.e. different genes that may originate from different species. Further gene activity regulating sequences are transcription factor binding elements (enhancers), wherein the transcription factor binding elements (enhancers) are operably linked to the nucleic acids used herein. Promoters and enhancers that may be used in nucleic acid constructs are known to the person skilled in the art and may be selected, e.g. depending on the type of tumour used, etc., with substantial information available for example via www.genetherapynet.com or common textbooks, e.g. Le Doux, J. (Ed.), Gene Therapy Protocols Vol. 1; Production and In vivo applications of Gene Transfer Vectors, Meth. Mol. Biol., Humana Press (2008), or Hunt, K.K. et al. (Eds.) Gene Therapy for Cancer (Cancer Drug Discovery and Development), Humana Press (2007).

The invention further provides a vector comprising a nucleic acid molecule according to the invention. In one embodiment, the vector is a viral vector. In another embodiment, the viral vector is a lentiviral vector, an adeno-associated virus-2 (AAV-2) vector, an adenoviral vector, a retroviral vector, a polio viral vector, a murine Muloney-based viral vector, an alpha viral vector, a pox viral vector, a herpes viral vector, a vaccinia viral vector, a baculoviral vector, a parvo viral vector, or any combination thereof. In one embodiment, a vector of the invention further comprises a carrier. In another embodiment, the carrier is a lipid. In another embodiment, the carrier is a polypeptide.

The nucleic acid molecules of the present invention are used to express US28 and at least one G-protein interacting with US28, or functional fragments of the polypeptides, or fusion proteins of the above polypeptides or functional fragments to induce apoptosis. Both nucleic acid molecules may be found on one vector or on separate vectors and these vectors, or compositions comprising the same, may be used contemporaneously or separately.

The G-protein according to the invention may be GNA13, which is an abbreviation for Guanine nucleotide-binding protein subunit alpha-13. This protein is encoded by the GNA13 gene (SEQ ID No. 3) on chromosome 17 in humans. In another embodiment, the G-protein may be GNA12, the gene encoding the same is found on human chromosome 7. The nucleotide sequence of this gene may be obtained from public databases such as GenBank, etc.

Nucleic acid constructs of the invention comprise a novel combination of genes which act together to induce apoptosis in cancer cells.

The invention provides nucleic acid molecules and the use thereof in methods of treating cancer, wherein said nucleic acid molecules comprise, consist essentially of or consist of a nucleotide sequence having at least about 50%, at least about 60%, at least about 70%, at least about 75%, at least about 80%, at least about 85%, at least about 90%, at least about 95%, at least about 99% or 100% identity to the nucleotide sequence encoding US28 and/or a G-protein (e.g. GNA13) interacting with US28, or functional fragments of such proteins that induce apoptosis in cancer cells. The nucleic acids may also comprise regulatory elements that control their expression, e.g. promoters, enhancers, ribosome entry sites, termination sequences, etc..

In certain embodiments, a nucleic acid encompassed by the invention is delivered to a cell. Methods for delivery of a nucleic acid to a cell in vitro or in vivo are known to those skilled in the art. For example, such methods may include the use of peptides, lipids or organic molecules as nucleic acid carriers to facilitate or enhance the cellular uptake of a nucleic acid. Nonlimiting examples of such nucleic acid delivery methods and nucleic carriers are described in U.S. Patent Nos. 6,344,436, 6,514,947, 6,670,177, 6,743,779, 6,806,084, and 6,903,077. The targeting of vehicles for the delivery of the nucleic acid molecules of the invention may be facilitated by the use of target cell specific molecules, e.g. receptors recognizing structures on the target cancer cells. These receptors may be immunoglobulin derived molecules.

### Pharmaceutical formulations and compositions

Pharmaceutical formulations or compositions comprising the nucleic acids of the invention include those suitable for parenteral (including intramuscular, subcutaneous and intravenous) administration. Forms suitable for parenteral administration also include forms suitable for administration by inhalation or insufflation or for nasal, or topical (including buccal, rectal, vaginal and sublingual) administration. The formulations may, where appropriate, be conveniently presented in discrete unit dosage forms and may be prepared by any of the methods well known in the art of pharmacy. Such methods include the step of bringing into association the active compound with liquid carriers, solid matrices, semi-solid carriers, finely divided solid carriers or combinations thereof, and then, if necessary, shaping the product into the desired delivery system.

The compositions or pharmaceutical formulations may be used once or frequently over a treatment period to achieve sufficiently strong therapeutic effects. The treatment may repeated upon verification of its efficacy using standard diagnostic measures.

The composition(s) disclosed herein and the nucleic acids of the invention may be used alone or in combination, i.e. the treatment of cancer may be effected by first administering a composition comprising either one nucleic acid of the invention in a separate composition (e.g. a composition comprising nucleic acids encoding US28 or a functional fragment thereof) and then administering a further composition comprising another nucleic acid of the invention in further composition (e.g. a composition comprising nucleic acids encoding GNA13 or a functional fragment thereof). The compositions may also be administered at the same time. When the compositions or nucleic acids are not co-administered, i.e. a sequence of administrations is used, the method of treatment may comprise one or more steps to determine that the administered nucleic acid is indeed expressed in tumour cells. Antibodies recognizing the expressed proteins may be used to confirm expression in the target cells, i.e. the cancer cells.

Alternatively, the compositions may comprise both, US28 and G-protein encoding nucleic acids of the invention. Compositions comprising each one of the nucleic acids of the invention may be administered separately, i.e. timely spaced, wherein the order of administration may be either way, i.e. in a first step a composition comprising a nucleic acid of the invention encoding US28 or a fragment thereof may be administered, and subsequently a composition comprising a nucleic acid encoding a G-protein, e.g. GNA13, or a functional fragment thereof is administered. The correct administration scheme may be determined by the medical staff. In some embodiments, the subject to be treated with the compositions described herein is a mammal. Nonlimiting examples of mammals include: humans, primates, mice, rabbits, rats, cats, and dogs.

### Further aspects of the invention

The present invention also relates to compositions comprising the above nucleic acid molecules for use as a medicament. The compositions may be used in the treatment of cancer. The compositions for use in the treatment according to the present invention are effective to treat cancer that may be selected from the group consisting of bladder cancer, bone cancer, brain cancer, cancer of other nervous tissues, breast cancer, cervical cancer, colon cancer, oesophagus cancer, eye cancer, gastrointestinal cancer, gynaecologic cancer, head and neck cancer, kidney cancer, laryngeal cancer, leukaemia, liver cancer, lung cancer, lymphoma, melanoma, mesothelioma, multiple myeloma, oral cancer, ovarian cancer, pancreatic cancer, prostate cancer, rectal cancer, renal cancer, skin cancer, stomach cancer, testicular cancer, throat cancer, thyroid cancer, uterine cancer, vaginal cancer, vulvar cancer. In one embodiment of the invention, the compositions and methods described herein are effective in the treatment of skin cancer, e.g. melanoma.

The compositions for use in the treatment referred to above may be used in combination with additional therapeutic means or methods in the treatment of cancer, for example those that are selected from the group comprising surgery, chemotherapy, radiation therapy, molecular cancer therapy or a further gene therapy, which may be used for administering genes that are different from the herein described nucleic acids of the invention.

A method of producing the composition referred to above is another aspect of the present invention. The method comprises the step of (a) producing the nucleic acid molecules of the invention, e.g. using standard cloning, multiplication and purification techniques, and (b) optionally formulation of the obtained nucleic acid(s) with suitable excipients for use in therapeutic applications.

The invention provides also a method for eliminating cancer cells in a subject in need thereof, comprising administering to the subject an effective amount of a nucleic acid or composition provided by the invention.

In one embodiment of the methods of the invention, the cancer cell may be a skin cancer cell, e.g. a melanoma cell, a basal cell carcinoma cell, a Merkel cell carcinoma cell, a squamous cell carcinoma cell, or cells from precursor lesions like actinic or solar keratosis, a breast cancer cell, a non-small cell lung cancer, a small cell lung cancer, colon cancer, or bladder cancer etc.

In one embodiment of the methods of the invention, the administration of an effective amount of the nucleic acid molecules, e.g. vectors of the invention, comprises parenteral, intralesional, intraperitoneal, intramuscular, intratumoral, subcutaneous, intraventricular, intracranial, intraspinal or intravenous injection; infusion; liposome- or vector-mediated delivery; or topical, nasal, oral, ocular, otic delivery, or any combination thereof.

The present invention also relates to a new gene therapy for cancer based on expression of novel vector constructs to selectively induce apoptosis in cancer cells. The construct can be delivered to cancer cells, for example, via a viral or non-viral vector. In some aspects of the invention, a nonviral vector may be used to facilitate delivery of a nucleic acid of the invention. The vectors may be formulated as pharmaceutical compositions.

In a further embodiment of the present invention, a method of transducing or transfecting a cell with the nucleic acid molecules referred to above is provided.

A cell that is transduced or transfected with the nucleic acid constructs referred to above forms another aspect of the present invention. Also transgenic animals expressing the above described nucleic acids, either separately or as fusion nucleic acid sequences, form an aspect of the present invention. The transgenes may be expressed in a tissue-specific manner. The expression of the transgenes may be conditionally and/or inducible.

The efficacy of the nucleic acids used according to the present invention in the induction of apoptosis may be determined using conventional methods for detection of apoptosis known to the person skilled in the art. Kits for the detection of apoptosis are available from various commercial sources.

The invention also provides a method for treating cancer in a subject in need thereof, or alternatively a composition as defined above for use in the treatment of tumours, wherein the treatment results in the tumour-specific induction of apoptosis in tumour cells avoiding side effects, associated with the destruction of cells that are not tumour cells.

Moreover, the present invention relates to methods for the detection of the herein described nucleic acids, e.g. a method for the detection of nucleic acids encoding fusion proteins. Such methods may be e.g. PCR methods using specific primers, hybridization methods using specific probes, etc., which are well known to persons skilled in the art.

Furthermore, the preparation of antibodies specifically recognizing novel fusion polypeptides or peptides encoded by the nucleic acids herein described is contemplated. The antibodies, e.g. monoclonal antibodies, fragments thereof, single chain antibodies etc., which may optionally be coupled with labels or compounds, e.g. toxins, radioactive substances, binding molecules, His-tails, FLAG-epitopes, etc., recognize epitopes that are specific for the fusion polypeptide, i.e. epitopes that are not present when the nucleic acid molecules encoding the fusion polypeptide(s) are expressed separately. These antibodies may be used to detect successful transduction or transfection and expression of the encoded polypeptides in a cell or organism. The person skilled in the art is familiar with methods for the preparation of such antibodies and with the use of antibodies to detect the presence of a protein of interest and to determine specific binding of such antibodies (cf. Harlow and Lane, Antibodies: A Laboratory Manual, Cold Spring Harbor Laboratory Press, Cold Spring Harbor, New York, 1988; Kontermann, R. & Dübel, S. (Eds.), Antibody Engineering Vol. 1 and Vol. 2, 2nd Ed. 2010, Springer Protocols, Springer).

In another aspect of the invention, gene therapeutic methods and/or the therapeutic use of the inventive nucleic acid constructs, or of compositions comprising the same, provides new means and methods for the induction of apoptosis in transfected or transduced cancer cells.

### EXAMPLES

The following examples illustrate the present invention, and are set forth to aid in the understanding of the invention, and should not be construed to limit in any way the scope of the invention as defined in the claims which follow thereafter.

### Example 1 US28 expression in melanoma cells leads to caspase mediated apoptosis and reduces proliferation

To determine the effect of expression of US28 in melanoma cells, 451Lu cells, a highly aggressive melanoma cell line, were transiently transfected with pcDNA3.1-GFP (Green Fluorescent Protein) and pcDNA3.1-US28 wild type (WT; US28 WT being depicted in SEQ ID NO: 1).

At 72 hrs post transfection the cells were harvested, fixed, and stained with Propidium iodide (PI) to determine apoptosis stimulated DNA fragmentation by fluorescent activated cell sorting (FACS). Measurements were performed on a BD FACS-Calibur flow cytometer. (B) In parallel total cell count was determined using CASY cell counter system. The results shown are representative of three independent experiments with p value < 0.01.

DNA fragmentation with an increase of about 12% in apoptotic cells for US28 expressing cells compared to control GFP transfected cells was observed. Considering the approximately 40-45% transfection efficiency with US28, the apoptotic cells correlate to about 25% cells undergoing apoptosis at 72 hrs post transfection (Fig. 1A). The cell count at 72 hrs indicates a 50% reduction in cell numbers for US28 transfected cells (Fig. 1B).

Induction of apoptosis was further confirmed by caspase activation and PARP cleavage by immunoblotting (Fig. 2). US28 induces caspase mediated apoptosis. 451Lu cells transfected with US28, control GFP and positive control BAX plasmids (the nucleotide sequence of BAX is depicted in SEQ ID NO: 7) were harvested at 72 hrs post transfection. Total protein (25 µg of each) was separated on SDS-PAGE, transferred to PVDF membrane and immunoblotted with antibodies against β-actin (Sigma), HA antibody for US28 tag (Covance), BAX antibody (eBiosciences), caspase 3 antibody (Santa Cruz biotechnology) and PARP antibody (eBiosciences) as per the recommended dilutions by the manufacturer, followed by respective secondary antibody and chemiluminescence detection. The arrows indicate the cleaved products due to activation of apoptotic pathways. There was a significant increase in activated caspase-3 on transfection with BAX or US28 (SEQ ID NO: 7 and SEQ ID NO: 1, respectively). An increase was also observed for the cleavage product of PARP, the indicator target of activated caspases, due to US28 expression detected at 72 hrs post-transfection.

### Example 2 US28 effects are dependent on its signaling activity

The constitutive signaling ability of US28 is suspected to lead to caspase dependent apoptosis. To confirm this and to determine the mechanism of US28, further experiments were performed with inclusion of a signaling mutant of US28, R129A, that lacks the constitutive signaling ability due to mutation at the second intracellular loop at 129 amino acid position 129A (Fig. 3). The nucleotide sequence of US28-R129A is shown in SEQ ID NO: 2.

Precisely, 451Lu cells transiently transfected with US28WT, US28R129A and control GFP plasmids analyzed at 72 hrs post transfection.

Figure 3(A) shows a comparative apoptotic DNA fragment analysis of cells transfected with GFP, WT and R129A that were harvested, fixed, and stained with PI and measurements were performed on BD FACS-Calibur flow cytometer.

Figure 3(B) shows the results of 451Lu melanoma cells in 96 well plates that were transfected, and analyzed at 72 hrs after transfection by MTT assay kit (ATCC) by a colorimetric method. Each construct was used in triplicates and analyzed against control GFP transfected cells for relative cell viability, p value < 0.01.

In Figure 3(C) the results of a cell count performed at 72 hrs using a CASY counter in 451Lu cells that have been transfected with respective plasmids are shown. The percent reduction in cell number was plotted against GFP control cells. The results shown are representative of three independent experiments, p value < 0.01.

As shown in Figure 3(D) during all these experiments the expression of US28 was confirmed by immunostaining procedure with HA primary antibody against US28 tag and detected with Alexa flour 488 secondary antibody. The surface levels of US28, both of the wildtype WT and the mutant form R129A are visible as green fluorescence at the plasma membrane.

The experiments determining total cell count and MTT assay along with FACS analysis for apoptosis at 72 hours post-transfection indicate that the signaling mutant has significantly lower apoptosis inducing ability.

US28 has been shown to interact with a wide number of hetero-trimeric G-protein complexes in a promiscuous manner. To determine as to whether the anti-proliferative/apoptotic effect of US28 is possibly associated with GNA13, 451Lu cells were silenced for expression of GNA13 using siRNA, followed by transfection with US28 WT and R129A constructs along with GFP control. The transfected cells were analyzed at 72 hrs after transfection by MTT assay kit (ATCC) and colorimetric measurements were performed. SD from triplicate readings for each set was calculated and analyzed against control GFP transfected cells for relative cell viability, p value < 0.01. A control set of scrambled siRNA silencing was performed in parallel. Reversal of the US28 effect in the case of GNA13 if compared to scrambled siRNA silencing experiments (Fig. 4A). Immunoblotting confirmed silencing of GNA13 as visualized by a nearly complete downregulation of GNA13 up to 96 hrs which was confirmed up to 96 hrs by separating total proteins on SDS-PAGE, transfer to PVDF membrane and immunoblotted for β-actin (Sigma) and GNA13 (Santa Cruz Biotechnology) protein levels (Fig. 4B).

A control set of scrambled siRNA silencing was performed in parallel. There was no significant change in cell growth properties due to silencing of GNA13 as determined by proliferation assay (data not shown).

As shown in Figure 5, the US28 effect on 451Lu cells is not affected by the absence of GNAQ (the nucleotide sequence of GNAQ is depicted in SEQ ID NO: 5). To demonstrate this, 451Lu cells before US28 transfection were transfected with siRNA for GNAQ along with control sc-siRNA. The silenced cells were transfected with GFP control, US28 WT and US28 R129A plasmids and analyzed at 72 hrs after transfection by MTT assay kit (ATCC) and colorimetric measurements were performed. SD from triplicate readings for each set was calculated and analyzed against control GFP transfected cells for relative cell viability, p value <0.01.

Furthermore, 451Lu cells were transfected with BAX as positive control and constructs with either GNA13 and GNAQ G-proteins and MTT assay performed at 72 hrs post-transfection. The results indicated that the GNA13 pathway is important for anti-proliferative effect. The GNAQ-mediated pathway does not seem to be of high importance, p value < 0.01.

Thus, the role of other potential G-protein complexes reportedly interacting with US28 towards similar activity in the experimental settings was ruled out by experiments involving either a G-protein pathway specific inhibitor (Adenylate cyclase inhibitor for GNAS G-protein (GNAQ siRNA; Figure 5).

Overall these results clearly show the importance of GNA13 in executing the apoptotic/anti-proliferative effect of US28 for melanoma cells. However, a possible involvement of other G-proteins, especially of Galpha12, in different target cell types cannot be ruled out.

## Claims

1. A nucleic acid molecule comprising a nucleotide sequence encoding US28 (SEQ ID NO: 1) or a functional fragment thereof and a nucleic acid molecule comprising a nucleotide sequence encoding a G-protein a functional fragment or a functional derivative thereof, and/or a nucleic acid molecule comprising a nucleotide sequence comprising a fusion of a nucleotide sequence encoding US28 (SEQ ID NO. 1) or a functional fragment thereof with a nucleotide sequence encoding a G-protein or functional fragment thereof for use in the treatment of cancer.

2. The nucleic acid molecule comprising a nucleotide sequence encoding US28 (SEQ ID NO: 1) or a functional fragment thereof and a nucleic acid molecule comprising a nucleotide sequence encoding a G-protein or a functional fragment, and/or a nucleic acid molecule comprising a nucleotide sequence comprising a fusion of a nucleotide sequence encoding US28 (SEQ ID NO. 1) or a functional fragment thereof with a nucleotide sequence encoding a G-protein or functional fragment thereof for use according to claim 1, wherein said nucleic acid molecules are for concomitant or separate administration.

3. A polypeptide encoded by a fusion of nucleic acid molecules comprising a nucleotide sequence encoding US28 (SEQ ID NO: 1) or a functional fragment thereof and a nucleotide sequence encoding a G-protein or a functional fragment thereof, wherein said G-protein optionally is GNA13 (SEQ ID NO: 3) or a functional fragment thereof.

4. A nucleic acid molecule comprising a nucleotide sequence encoding a polypeptide according to claim 3.

5. The nucleic acid molecules comprising a nucleotide sequence encoding a G-protein or a functional fragment thereof for use according to claims 1, 2 or 4, wherein the G-protein is GNA13 (SEQ ID NO: 3) or a functional fragment thereof.

6. A pharmaceutical composition comprising:
(i) a nucleic acid molecule comprising a nucleotide sequence encoding US28 (SEQ ID NO: 1) or a functional fragment thereof, and
(ii) a nucleic acid molecule comprising a nucleotide sequence encoding a G-protein or a functional fragment thereof.

7. A pharmaceutical composition comprising the nucleic acid sequence molecule according to claim 4 or claim 5.

8. The pharmaceutical composition according to any of claims 6 or 7, wherein the nucleotide sequences encoding a G-protein or a functional fragment thereof are GNA13 (SEQ ID NO: 3) or a functional fragment thereof.

9. The pharmaceutical composition according to any of claims 6 to 8 for use in the treatment and/or prevention of cancer.

10. The pharmaceutical composition for use according to claim 9, wherein the cancer is selected from the group consisting of skin cancer, melanoma, basal cell carcinoma cell, Merkel cell carcinoma cell, squamous cell carcinoma cell, actinic or solar keratosis, breast cancer cell, non-small cell lung cancer, small cell lung cancer, colon cancer or bladder cancer.

11. A kit comprising the nucleic acid molecule(s) as defined in claims 1, 2, 4 or 5, or the pharmaceutical compositions defined in claims 6 to 10.

12. The nucleic acid molecule for use according to claim 1 or 2, or the pharmaceutical composition as defined in any of claims 6 to 10 for use in a combination therapy with at least one additional therapy selected from the group comprising surgery, chemotherapy, radiation therapy, molecular cancer therapy, and cancer gene therapy in the treatment of cancer.

13. A method of producing the nucleic acid molecules, polypeptide, pharmaceutical compositions and kits as defined in the preceding claims.

14. A method of transfecting or transducing a cell with the nucleic acid molecules as defined in claims 1, 2, 4 or 5.

15. A cell or organism that is transfected or transduced with the composition according to any of the preceding claims.

16. An antibody specifically recognizing the polypeptide of claim 3.


SEQUENCE LISTING

<110>  Medizinische Universität Graz

<120>  Cancer gene  therapy using US28 and G-Protein

<130>  M 10026 / DJB

<160>  7

<170>  PatentIn version 3.3

<210>  1
<211>  1095
<212>  DNA
<213>  Human Cytomegalovirus


<220>
<221>  misc_feature
<223>  US28 Wildtype

<400>  1
atgggctacc cgtacgacgt cccagactac gccacaccga cgacgacgac cgcggaactc      60

acgacggagt ttgactacga cgatgaagcg actccctgtg tcctcaccga cgtgcttaat     120

cagtcgaagc cagtcacgtt gtttctgtac ggcgttgtct ttctcttcgg ttccatcggc     180

aacttcttgg tgatcttcac catcacctgg cgacgtcgga ttcaatgttc cggcgatgtt     240

tactttatca acctcgcggc cgccgatttg cttttcgttt gtacactacc tctgtggatg     300

caatacctcc tagatcacaa ctccctagcc agcgtgccgt gtacgttact cactgcctgt     360

ttctacgtgg ctatgtttgc cagtttgtgt tttatcacgg agattgcact cgatcgctac     420

tacgctattg tttacatgag atatcggcct gtaaaacagg cctgcctttt cagtattttt     480

tggtggatct ttgccgtgat catcgccatt ccacacttta tggtggtgac caaaaaagac     540

aatcaatgta tgaccgacta cgactactta gaggtcagtt acccgatcat cctcaacgta     600

gaactcatgc tcggtgcttt cgtgatcccg ctcagtgtca tcagctactg ctactaccgc     660

atttccagaa tcgttgcggt gtctcagtcg cgccacaaag gccgcattgt acgggtactt     720

atagcggtcg tgcttgtctt tatcatcttt tggctgccgt accacctgac gctgtttgtg     780

gacacgttga aactgctcaa atggatctcc agcagctgcg agttcgaaaa atcactcaag     840

cgcgcgctca tcttgaccga gtcactcgcc ttttgtcact gttgtctcaa tccgctgctg     900

tacgtcttcg tgggcaccaa gtttcggcaa gaactgcact gtctgctggc cgagtttcgc     960

cagcgactgt tttcccgcga tgtatcctgg taccacagca tgagcttttc gcgtcggagc    1020

tcgccgagcc gaagagagac gtcttccgac acgctgtccg acgaggcgtg tcgcgtctca    1080

caaattatac cgtaa                                                     1095


<210>  2
<211>  1095
<212>  DNA
<213>  Human CMV


<220>

<221>  misc_feature
<223>  US28 mutant A129

<400>  2
atgggctacc cgtacgacgt cccagactac gccacaccga cgacgacgac cgcggaactc      60

acgacggagt ttgactacga cgatgaagcg actccctgtg tcctcaccga cgtgcttaat     120

cagtcgaagc cagtcacgtt gtttctgtac ggcgttgtct ttctcttcgg ttccatcggc     180

aacttcttgg tgatcttcac catcacctgg cgacgtcgga ttcaatgttc cggcgatgtt     240

tactttatca acctcgcggc cgccgatttg cttttcgttt gtacactacc tctgtggatg     300

caatacctcc tagatcacaa ctccctagcc agcgtgccgt gtacgttact cactgcctgt     360

ttctacgtgg ctatgtttgc cagtttgtgt tttatcacgg agattgcact cgatgcatac     420

tacgctattg tttacatgag atatcggcct gtaaaacagg cctgcctttt cagtattttt     480

tggtggatct ttgccgtgat catcgccatt ccacacttta tggtggtgac caaaaaagac     540

aatcaatgta tgaccgacta cgactactta gaggtcagtt acccgatcat cctcaacgta     600

gaactcatgc tcggtgcttt cgtgatcccg ctcagtgtca tcagctactg ctactaccgc     660

atttccagaa tcgttgcggt gtctcagtcg cgccacaaag gccgcattgt acgggtactt     720

atagcggtcg tgcttgtctt tatcatcttt tggctgccgt accacctgac gctgtttgtg     780

gacacgttga aactgctcaa atggatctcc agcagctgcg agttcgaaaa atcactcaag     840

cgcgcgctca tcttgaccga gtcactcgcc ttttgtcact gttgtctcaa tccgctgctg     900

tacgtcttcg tgggcaccaa gtttcggcaa gaactgcact gtctgctggc cgagtttcgc     960

cagcgactgt ttcccgcgat tgtatcctgg taccacagca tgagcttttc gcgtcggagc    1020

tcgccgagcc gaagagagac gtcttccgac acgctgtccg acgaggcgtg tcgcgtctca    1080

caaattatac cgtaa                                                     1095

<210>  3
<211>  1134
<212>  DNA
<213>  human

<220>
<221>  misc_feature
<223>  GNA13

<400>  3
atggcggact tcctgccgtc gcggtccgtg ctgtccgtgt gcttccccgg ctgcctgctg      60

acgagtggcg aggccgagca gcaacgcaag tccaaggaga tcgacaaatg cctgtctcgg     120

gaaaagacct atgtgaagcg gctggtgaag atcctgctgc tgggcgcggg cgagagcggc     180

aagtccacct tcctgaagca gatgcggatc atccacgggc aggacttcga ccagcgcgcg     240

cgcgaggagt tccgccccac catctacagc aacgtgatca aaggtatgag ggtgctggtt     300

gatgctcgag agaagcttca tattccctgg ggagacaact caaaccaaca acatggagat     360

aagatgatgt cgtttgatac ccgggccccc atggcagccc aaggaatggt ggaaacaagg     420

gttttcttac aatatcttcc tgctataaga gcattatggg cagacagcgg catacagaat     480

gcctatgacc ggcgtcgaga atttcaactg ggtgaatctg taaaatattt cctggataac 540

ttggataaac ttggagaacc agattatatt ccatcacaac aagatattct gcttgccaga 600

agacccacca aaggcatcca tgaatacgac tttgaaataa aaaatgttcc tttcaaaatg 660

gttgatgtag gtggtcagag atcagaaagg aaacgttggt ttgaatgttt cgacagtgtg 720

acatcaatac ttttccttgt ttcctcaagt gaatttgacc aggtgcttat ggaagatcga 780

ctgaccaatc gccttacaga gtctctgaac atttttgaaa caatcgtcaa taaccgggtt 840

ttcagcaatg tctccataat tctgttctta aacaagacag acttgcttga ggagaaggtg 900

caaattgtga gcatcaaaga ctatttccta gaatttgaag gggatcccca ctgcttaaga 960

gacgtccaaa aattcctggt ggaatgtttc cggaacaaac gccgggacca gcaacagaag 1020

cccttatacc accacttcac cactgctatc aacacggaga acatccgcct tgttttccgt 1080

gacgtgaagg atactattct gcatgacaac ctcaagcagc ttatgctaca gtga 1134

<210> 4
<211> 1134
<212> DNA
<213> human

<220>
<221> misc_feature
<223> GNA13 QL

<400> 4
atggcggact tcctgccgtc gcggtccgtg ctgtccgtgt gcttccccgg ctgcctgctg 60

acgagtggcg aggccgagca gcaacgcaag tccaaggaga tcgacaaatg cctgtctcgg 120

gaaaagacct atgtgaagcg gctggtgaag atcctgctgc tgggcgcggg cgagagcggc 180

aagtccacct tcctgaagca gatgcggatc atccacgggc aggacttcga ccagcgcgcg 240

cgcgaggagt tccgccccac catctacagc aacgtgatca aaggtatgag ggtgctggtt 300

gatgctcgag agaagcttca tattccctgg ggagacaact caaaccaaca acatggagat 360

aagatgatgt cgtttgatac ccgggccccc atggcagccc aaggaatggt ggaaacaagg 420

gttttcttac aatatcttcc tgctataaga gcattatggg cagacagcgg catacagaat 480

gcctatgacc ggcgtcgaga atttcaactg ggtgaatctg taaaatattt cctggataac 540

ttggataaac ttggagaacc agattatatt ccatcacaac aagatattct gcttgccaga 600

agacccacca aaggcatcca tgaatacgac tttgaaataa aaaatgttcc tttcaaaatg 660

cttgatgtag gtggcctgag gtcagaaagg aaacgttggt ttgaatgttt cgacagtgtg 720

acatcaatac ttttccttgt ttcctcaagt gaatttgacc aggtgcttat ggaagatcga 780

ctgaccaatc gccttacaga gtctctgaac atttttgaaa caatcgtcaa taaccgggtt 840

ttcagcaatg tctccataat tctgttctta aacaagacag acttgcttga ggagaaggtg 900

caaattgtga gcatcaaaga ctatttccta gaatttgaag gggatcccca ctgcttaaga 960

gacgtccaaa aattcctggt ggaatgtttc cggaacaaac gccgggacca gcaacagaag 1020

cccttatacc accacttcac cactgctatc aacacggaga acatccgcct tgttttccgt 1080

gacgtgaagg atactattct gcatgacaac ctcaagcagc ttatgctaca gtga 1134

<210> 5
<211> 1080
<212> DNA
<213> human

<220>
<221> misc_feature
<223> GNAQ

<400> 5
atgactctgg agtccatcat ggcgtgctgc ctgagcgagg aggccaagga agcccggcgg 60

atcaacgacg agatcgagcg gcagctccgc agggacaagc gggacgcccg ccgggagctc 120

aagctgctgc tgctcgggac aggagagagt ggcaagagta cgtttatcaa gcagatgaga 180

atcatccatg ggtcaggata ctctgatgaa gataaaaggg gcttcaccaa gctggtgtat 240

cagaacatct tcacggccat gcaggccatg atcagagcca tggacacact caagatccca 300

tacaagtatg agcacaataa ggctcatgca caattagttc gagaagttga tgtggagaag 360

gtgtctgctt ttgagaatcc atatgtagat gcaataaaga gtttatggaa tgatcctgga 420

atccaggaat gctatgatag acgacgagaa tatcaattat ctgactctac caaatactat 480

cttaatgact tggaccgcgt agctgaccct gcctacctgc ctacgcaaca agatgtgctt 540

agagttcgag tccccaccac agggatcatc gaatacccct ttgacttaca aagtgtcatt 600

ttcagaatgg tcgatgtagg gggccaaagg tcagagagaa gaaaatggat acactgcttt 660

gaaaatgtca cctctatcat gtttctagta gcgcttagtg aatatgatca agttctcgtg 720

gagtcagaca atgagaaccg aatggaggaa agcaaggctc tctttagaac aattatcaca 780

tacccctggt tccagaactc ctcggttatt ctgttcttaa acaagaaaga tcttctagag 840

gagaaaatca tgtattccca tctagtcgac tacttcccag aatatgatgg accccagaga 900

gatgcccagg cagcccgaga attcattctg aagatgttcg tggacctgaa cccagacagt 960

gacaaaatta tctactccca cttcacgtgc gccacagaca ccgagaatat ccgctttgtc 1020

tttgctgccg tcaaggacac catcctccag ttgaacctga aggagtacaa tctggtctaa 1080

<210> 6
<211> 1080
<212> DNA
<213> human

<220>
<221> misc_feature
<223> GNA QL

<400> 6
atgactctgg agtccatcat ggcgtgctgc ctgagcgagg aggccaagga agcccggcgg 60

atcaacgacg agatcgagcg gcagctccgc agggacaagc gggacgcccg ccgggagctc 120

aagctgctgc tgctcgggac aggagagagt ggcaagagta cgtttatcaa gcagatgaga 180

atcatccatg ggtcaggata ctctgatgaa gataaaaggg gcttcaccaa gctggtgtat 240

cagaacatct tcacggccat gcaggccatg atcagagcca tggacacact caagatccca 300

tacaagtatg agcacaataa ggctcatgca caattagttc gagaagttga tgtggagaag 360

gtgtctgctt ttgagaatcc atatgtagat gcaataaaga gtttatggaa tgatcctgga 420

atccaggaat gctatgatag acgacgagaa tatcaattat ctgactctac caaatactat 480

cttaatgact tggaccgcgt agctgaccct gcctacctgc ctacgcaaca agatgtgctt 540

agagttcgag tccccaccac agggatcatc gaatacccct ttgacttaca aagtgtcatt 600

ttcagaatgg tcgatgtagg gggcctaagg tcagagagaa gaaaatggat acactgcttt 660

gaaaatgtca cctctatcat gtttctagta gcgcttagtg aatatgatca agttctcgtg 720

gagtcagaca atgagaaccg aatggaggaa agcaaggctc tctttagaac aattatcaca 780

tacccctggt tccagaactc ctcggttatt ctgttcttaa acaagaaaga tcttctagag 840

gagaaaatca tgtattccca tctagtcgac tacttcccag aatatgatgg accccagaga 900

gatgcccagg cagcccgaga attcattctg aagatgttcg tggacctgaa cccagacagt 960

gacaaaatta tctactccca cttcacgtgc gccacagaca ccgagaatat ccgctttgtc 1020

tttgctgccg tcaaggacac catcctccag ttgaacctga aggagtacaa tctggtctaa 1080

<210> 7
<211> 579
<212> DNA
<213> Homo sapiens

<220>
<221> misc_feature
<223> BAX

<400> 7
atggacgggt ccggggagca gcccagaggc gggggggcca ccagctctga gcagatcatg 60

aagacagggg cccttttgct tcagggtttc atccaggatc gagcagggcg aatggggggg 120

gaggcacccg agctggccct ggacccggtg cctcaggatg cgtccaccaa gaagctgagc 180

gagtgtctca agcgcatcgg ggacgaactg gacagtaaca tggagctgca gaggatgatt 240

gccgccgtgg acacagactc cccccgagag gtctttttcc gagtggcagc tgacatgttt 300

tctgacggca acttcaactg gggccgggtt gtcgcccttt tctactttgc cagcaaactg 360

gtgctcaagg ccctgtgcac caaggtgccg gaactgatca gaaccatcat gggctggaca 420

ttggacttcc tccgggagcg gctgttgggc tggatccaag accagggtgg ttgggacggc 480

ctcctctcct actttgggac gcccacgtgg cagaccgtga ccatctttgt ggcgggagtg 540

ctcaccgcct cactcaccat ctggaagaag atgggctaa 579